(19) Europäisches Patentamt
European Patent Office
Office européen des brevets

(11) Veröffentlichungsnummer: **0 264 865 A2**

(12) **EUROPÄISCHE PATENTANMELDUNG**

(21) Anmeldenummer: **87115217.9**

(22) Anmeldetag: **17.10.87**

(51) Int. Cl.⁴: **C07D 211/60**, C07D 279/06, C07D 279/10, A01N 43/34, C07D 401/06

(30) Priorität: **24.10.86 DE 3636278**

(43) Veröffentlichungstag der Anmeldung: **27.04.88 Patentblatt 88/17**

(84) Benannte Vertragsstaaten: **AT BE CH DE FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Frey, Michael, Dr.**
**Meraner Strasse 26a**
**D-8902 Neusäss(DE)**
Erfinder: **Ehrhardt, Heinz, Dr.**
**Bergstrasse 21**
**D-8901 Rehling(DE)**
Erfinder: **Mildenberger, Hilmar, Dr.**
**Fasanenstrasse 24**
**D-6233 Kelkheim (Taunus)(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein(DE)**

(54) **Alpha-Iminocarbonsäureanilide enthaltende herbizide Mittel sowie neue Alpha-Iminocarbonsäureanilide und Verfahren zu ihrer Herstellung.**

(57) Herbizide Mittel, die eine oder mehrere Verbindungen der Formel I oder deren Salze

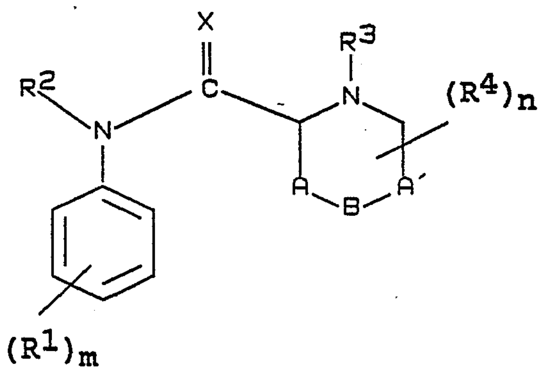

(I)

worin
A,A' = $CH_2$, S, $CHR^4$, $CR^4_2$, SO oder $SO_2$; B = O, S, SO, $SO_2$, $CH_2$, $CHR^4$ $CR^4_2$, $NR^5$, $NCXR^5$ oder $NCX_2R^5$;
X = O oder S; $R^1$ = Hal, $NO_2$, XH, (subst.) Alkyl(oxy oder mercapto), Cycloalkyl(oxy oder mercapto),

EP 0 264 865 A2

Alkenyl(oxy oder mercapto), Cycloalkenyl(oxy oder mercapto), Alkinyl(oxy oder mercapto), (subst.) Alkoxycarbonyl(oxy oder mercapto), Alkoxycarbonylmethylamino; $R^2$ = H oder (Hydroxy)Alkyl;
$R^3$ = H, (subst.)Alkyl, Cycloalkyl, Alkoxycarbonylmethyl, $R^5$, Nicotinoyl, 1-Imidazolylcarbonyl oder einen Rest der Formeln-C = X(Z),-CO-COZ, $-SOR^6$, $SO_2R^6$, $SO_2NHR^5$, $SO_2N(R^5)_2$, $PX(R^7)_2$ oder $-CO-CH_2-PX(R^7)_2$; $R^4$ = die Bedeutung von $R^5$, $CXR^5$ oder $CX_2R^5$; m = 0, 1, 2, 3, 4 oder 5; n = 0, 1, 2 oder 3 bedeuten, als Wirkstoff(e) enthalten, eignen sich hervorragend zur Bekämpfung von mono-oder dikotylen Schadpflanzen.

## α-Iminocarbonsäureanilide enthaltende herbizide Mittel sowie neue α-Iminocarbonsäureanilide und Verfahren zu ihrer Herstellung

Es wurde gefunden, daß die cyclischen α-Iminocarbonsäureanilide der nachfolgenden Formel I eine ausgezeichnete herbizide Wirkung aufweisen. Einige dieser Verbindungen sind aus Tetrahedron Letters 22 (1981) 2411, Acta Chim. Scand. 11 (1957) 1183 und JP-A 78-31, 669 bekannt. Über eine biologische Wirksamkeit dieser Verbindungen wurde jedoch nicht berichtet.

Gegenstand der vorliegenden Erfindung sind daher herbizide Mittel enthaltend mindestens eine Verbindung der Formel I oder deren Salz

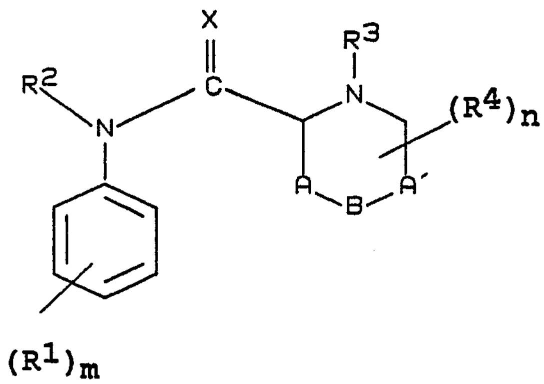

(I)

worin

A,A' = unabhängig voneinander $CH_2$, S, oder einer der beiden Reste A, A' = $CHR^4$, $C(R^4)_2$, >SO oder >$SO_2$,

B = O, S,>SO,>$SO_2$, $CH_2$, $CHR^4$, $C(R^4)_2$,>$NR^5$,>N-C=X($R^5$) oder > N-C=X($XR^5$), mit der Maßgabe, daß A und B oder A' und B nicht gleichzeitig O, S, SO oder $SO_2$ bedeuten dürfen,

X = O oder S,

$R_1$ = Halogen, $NO_2$, ($C_1$-$C_4$)Alkyl, -X($C_1$-$C_4$)Alkyl oder ($C_1$-$C_8$)Alkoxycarbonyl, wobei die letzten drei Reste bis zu sechsfach durch Halogen oder
ein-oder zweifach durch OH substituiert sein können,
($C_3$-$C_6$)Cycloalkyl, ($C_3$-$C_6$)-Alkenyl,
($C_5$-$C_6$)-Cycloalkenyl, ($C_3$-$C_7$)Alkinyl,
($C_1$-$C_4$)Alkoxy($C_1$-$C_4$)alkyl,
($C_1$-$C_4$)Alkoxy($C_1$-$C_4$)alkoxycarbonyl,
($C_1$-$C_8$)Alkoxycarbonyl($C_1$-$C_8$)-alkoxycarbonyl,
($C_1$-$C_8$)Alkoxycarbonylmethylamino, -XH,
-X($C_3$-$C_6$)Cycloalkyl, -X($C_3$-$C_6$)Alkenyl, -X($C_5$-$C_6$)-Cycloalkenyl, -X($C_3$-$C_7$)Alkinyl, -X[($C_1$-$C_4$)Alkoxy-($C_1$-$C_4$)alkyl] oder -X[($C_1$-$C_8$)Alkoxycarbonyl],

$R^2$ = H, ($C_1$-$C_4$)Alkyl oder ($C_1$-$C_4$)Hydroxyalkyl,

$R^3$ = H, ($C_1$-$C_6$)Alkyl, ($C_3$-$C_6$)Cycloalkyl, ($C_1$-$C_4$)Haloalkyl, ($C_1$-$C_4$)Alkoxy($C_1$-$C_4$)alkyl, ($C_1$-$C_4$)-Alkoxycarbonylmethyl, Phenoxy($C_1$-$C_4$)alkyl, Nicotinoyl, 1-Imidazolylcarbonyl, die Bedeutung von $R^5$ oder einen Rest der Formel

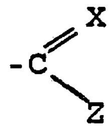

oder 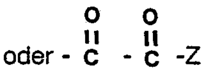 , worin

Z = die Bedeutung von $R^5$ besitzt oder den Rest $OR^5$, Halogen($C_1$-$C_4$)alkyl, ($C_1$-$C_4$)Alkoxy($C_1$-$C_4$)alkyl, Phenoxy($C_1$-$C_4$)alkyl, ($C_1$-$C_4$)Alkylamino oder Phenylamino, worin der Phenylrest durch Halogen, ($C_1$-$C_4$)-Alkoxy oder ($C_1$-$C_4$)Alkyl substituiert sein kann, bedeutet,

ferner einen Rest der Formeln

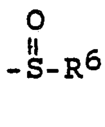 , 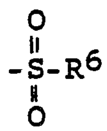 , 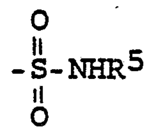 , 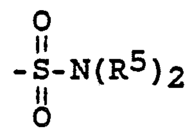 , 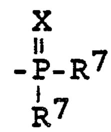

oder 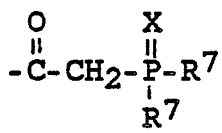 ,

$R^4$ = unabhängig voneinander $R^5$, $-C=X(R^5)$ oder $-C=X(XR^5)$,

$R^5$ = Phenyl, Pyridyl oder Benzyl,die bis zu dreifach durch ($C_1$-$C_4$)Alkyl, ($C_1$-$C_4$)Alkoxy, Halogen, $CF_3$ oder $NO_2$ substituiert sein können, oder ($C_1$-$C_8$)Alkyl,

$R^6$ = unabhängig voneinander H, Halogen, OH, die Bedeutung von $R^5$ oder den Rest $OR^5$,

$R^7$ = die Bedeutung von $R^6$ sowie SH oder den Rest $SR^5$,

m = 0, 1, 2, 3, 4 oder 5

n = 0, 1, 2 oder 3

bedeuten.

Im vorstehenden bedeutet Halogen vorzugweise F, Cl oder Br. ($C_1$-$C_4$)Haloalkyl steht beispielsweise für die Reste $-CF_3$, $-CH_2F_3$, $-CHF-CF_3$, $-CF_2-CF_2Cl$, $-CF_2-CF_2H$, $-CF_2-CHFCl$ oder $-CF_2-CHF-CF_3$. Halogeniertes -X-($C_1$-$C_4$)Alkyl steht insbesondere für $OCF_3$, $OCH_2-CF_3$, $-OCHF-CF_3$, $-OCF_2CHF_2$ oder $-OCF_2-CHF-CF_3$. Im Falle Z = Halogen($C_1$-$C_4$)alkyl sind Monochloralkyl-Reste bevorzugt, bei denen das Chloratom in Positon C-1 des Alkylrestes orientiert ist.

Die obengenannte Formel I umfaßt auch alle optischen Isomeren und Isomerengemische der entsprechenden Verbindungen sowie deren landwirtschaftlich verträgliche Salze. Die Salzbildung kann an einem Stickstoffatom des Heterocyclenteils erfolgen oder an der Carbonsäuregruppe im Falle $R^4$ = COOH. Als Salze kommen die in der Landwirtschaft üblichen Salze in Betracht. z. B. Alkali-, Erdalkali-, Ammonium-oder substituierte Ammoniumsalze.

Die Verbindungen der Formel I ausgenommen solche, bei denen

a) A, B und A' = $CH_2$; X = O; $R^1$ = Chlor, Methyl, Ethyl, Ethoxy, n-Butoxy; $R^2$ = H; $R^3$ = Methyl, Ethyl, n-Propyl oder n-Butyl, $(R^4)_n$ = H und m = 0, 1, 2 oder 3;

b) A, B und A' = $CH_2$; X = O; $R^2$ = H oder $C_2H_5$; $R^3$ = $CH_3$ oder $CO_2CH_3$; $(R^4)_n$ = H oder mono-$CH_3$ und m = O;

c) A, B und A' = $CH_2$; X = O; $(R^1)_m$ = 4-Chlor; $R^2$ = H; $R^3$ = nicotinoyl und $(R^4)_n$ = H; und

d) A und A' = $CH_2$; B = O; X = O; $(R^1)_m$ = H; $R^2$ = H; $R^3$ = $CO_2CH_3$ und $(R^4)_n$ = H gilt, sind neu. Diese neuen Verbindungen und deren Salze sind daher gleichfalls Gegenstand der Erfindung.

Als Reste der Formel

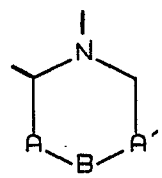

kommen bevorzugt in Frage der Piperidin-, Piperazin-, Morpholin-, Tetrahydro-1,4-thiazin-oder Tetrahydro-1,3-thiazin-Ring, die entweder unsubstituiert oder in der oben angegebenen Weise substituiert sein können sowie gegebenenfalls deren Sulfoxide oder Sulfone, wobei solche Reste, für die n = 0 ist, besonders bevorzugt sind.

Unter den Verbindungen der Formel I sind solche Verbindungen hervorzuheben, bei denen bedeuten: A, A', B = $CH_2$; X = O; $R^1$ = Halogen, $(C_1$-$C_4)$Alkoxy, $(C_3$-$C_7)$Alkinyloxy oder $(C_1$-$C_8)$Alkoxycarbonyl$(C_1$-$C_8)$-alkoxycarbonyl; $R^2$ = H, $(C_1$-$C_4)$Alkyl oder $-CH_2OH$; $R^3$ = $(C_1$-$C_4)$Alkoxycarbonyl, $(C_1$-$C_4)$Alkoxysulfonyl oder Di $(C_1$-$C_4)$Alkoxyphosphoryl, insbesondere $(C_1$-$C_4)$Alkoxycarbonyl; m = 1, 2 oder 3 und n = 0.

Im Anilinrest der Verbindungen der Formel I ist für den Rest $R^1$ die Substitution in Position 2, 4 oder 5 bevorzugt.

Gegenstand der vorliegenden Erfindung ist ferner auch ein Verfahren zur Herstellung der Verbindungen der Formel I oder deren Salze, wobei ein α-Iminocarbonsäureanilid der Formel II mit einer Verbindung der Formel III in Gegenwart einer Base umgesetzt wird und die erhaltene Verbindung gegebenenfalls auf übliche Weise in ihr Salz überführt wird.

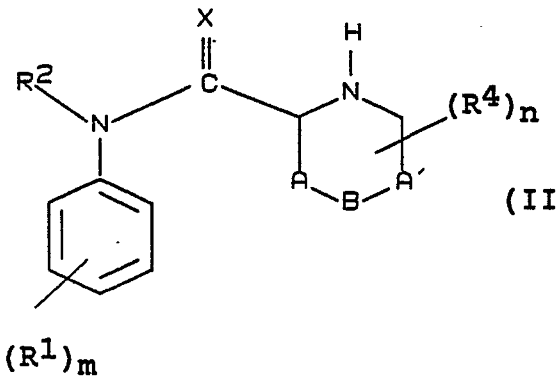

(II)

$R^3$-Hal

(III)

Die Umsetzung wird in einem inerten organischen Lösungsmittel wie Diethylether, Toluol oder Acetonitril bei Temperaturen von 0 - 80°C, vorzugsweise bei 20 - 40°C durchgeführt. Als Base können tertiäre Amine wie Triethylamin oder Pyridin oder anorganische Basen wie Alkalihydroxid oder Alkalicarbonat eingesetzt werden.

Die α-Iminocarbonsäureanilide der Formel II werden durch Umsetzung eines α-Iminocarbonsäurehydrochlorids mit einem Anilin der Formel

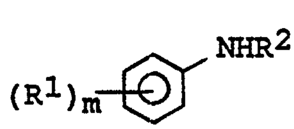

in Anlehnung an das von B. af Ekenstam (Acta Chim. Scand. 11, 1183 (1957)) beschriebende Verfahren hergestellt.

Die erfindungsgemäßen Verbindungen der Formel I weisen eine ausgezeichnete herbizide Wirksamkeit gegen ein breites Spektrum wirtschaftlich wichtiger mono-und dikotyler Schadpflanzen auf wie z.B. Avena fatua, Alopecurus, Setaria, Digitaria, Echinochloa, Sorghum, Imperata, Acropyron, Galium, Amaranthus, Abutilon, Ipomoea, Sinapis, Viola, Lamium, Veronica, Cirsium, Rumex, Sesbania. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono-und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann.

Die erfindungsgemäßen Verbindungen können, gegebenenfalls im Gemisch mit weiteren Wirkkomponenten, als Spritzpulver, emulgierbare Konzentrate, versprühbare Lösungen, Beizmittel, Stäubemittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs-oder Inertstoff oder Netzmittel wie polyoxyethylierte Alkylphenole, polyoxethylierte Fettalkohole, Alkyl-oder Alkylphenylsulfonate und/oder Dispergierhilfsmittel wie ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen der Wirkstoffe in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Kondensationsprodukte, Alkylpolyglykolether, Sorbitanfettsäureester, Polyoxethylensorbitansäureester oder Polyoxethylensorbitester.

Stäubemittel kann man durch Vermahlen der Wirkstoffe mit feinverteilten, festen Stoffen, z.B. Talkum, natürlichen Tonen wie Kaolin, Bentonit, Pyrophillit oder Diatomeenerde erhalten.

Granulate können entweder durch Verdüsen der Wirkstoffe auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 0,05 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösemittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen der Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt:

**Formulierungsbeispiele**

Beispiel 1

Ein Stäubemittel wird erhalten, indem man 10 Gewichtsteile Wirkstoff mit 90 Gewichtsteilen Talkum oder einem anderen Inertstoff mischt und in einer Schlagmühle zerkleinert.

Beispiel 2

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

Beispiel 3

Ein in Wasser leicht dispergierbares Dispersionskonzentrat wird erhalten indem man 20 Gewichtsteile Wirkstoff mit 6 Gewichtsteilen Alkylphenolpolyglykolether (®Triton X 207), 3 Gewichtsteilen Isotridecanolpolyglykolether (8 EO ) und 71 Gewichtsteilen paraffinischem Mineralöl (Siedebereich ca. 255 bis über 377°C) mischt und in einer Reibkugelmühle auf eine Feinheit von unter 5 Mikron vermahlt.

Beispiel 4

Ein emulgierbares Konzentrat wird erhalten aus 15 Gewichtsteilen Wirkstoff(e), 75 Gewichtsteilen Cyclohexanon als Lösemittel und 10 Gewichtsteilen oxethyliertem Nonylphenol (10 EO ) als Emulgator.

**Chemische Beispiele**

**Beispiel 1**

1-Ethoxycarbonylpiperidin-2-carbonsäure-(2-fluor-4-chlor-5-methoxy)anilid

Zu einer Lösung von 3,6 g (13 mmol) Piperidin-2-carbonsäure-(2-chlor-4-fluor-5-methoxy)anilid und 2,7 g (27 mmol) Triethylamin in 70 ml Toluol wird unter Rühren bei Raumtemperatur eine Lösung von 1,5 g (14 mmol) Chlorameisensäureethylester in 30 ml Toluol getropft. Man rührt 1 h bei Raumtemperatur und 2 h bei 50°C nach, saugt vom ausgefallenen Niederschlag ab und wäscht die Lösung zweimal mit halbkonzentrierter Salzsäure und zweimal mit Wasser. Man trocknet über Natriumsulfat, destilliert das Lösungsmittel ab

und trocknet den Rückstand im Vakuum. Man erhält 4,0 g (86 Gew.-%) 1-Ethoxycarbonylpiperidin-2-carbonsäure-(2-fluor-4-chlor-5-methoxy)anilid als hellgelbes, zähes Öl.

## Beispiel 2

1-Diethoxyphosphoryl-piperidin-2-carbonsäure-(4-chlor)anilid

Zu einer Lösung von 3,47 g (14,5 mmol) Piperidin-2-carbonsäure-(4-chlor)anilid und 2,93 g (29 mmol) Triethylamin in 40 ml Acetonitril wird unter Rühren eine Lösung von 2,50 g (14,5 mmol) Diethylchlorophosphat in 30 ml Acetonitril getropft. Man rührt drei Stunden bei 40°C nach und gießt das Gemisch in Eiswasser (dreifache Volumen).

Man extrahiert mit 3 × 100 ml Methylenchlorid, wäscht die vereinigten organischen Auszüge mit 2 × 50 ml 2 n HCl und 2 × 50 ml Wasser und trocknet über Natriumsulfat. Nach dem Abdestillieren des Lösungsmittels, dem Verreiben des Rückstands mit Diisopropylether, dem Absaugen und anschließendem Trocknen erhält man 3,80 g (70 Gew.-%) 1-Diethoxyphosphoryl-piperidin-2-carbonsäure-(4-chlor)anilid als hellbraunes Pulver (Schmelzpunkt 169 - 172°C).

EO: Ethylenoxid-Einheiten

EO: Ethylenoxid-Einheiten

Tabelle 1 Verbindungen der Formel I

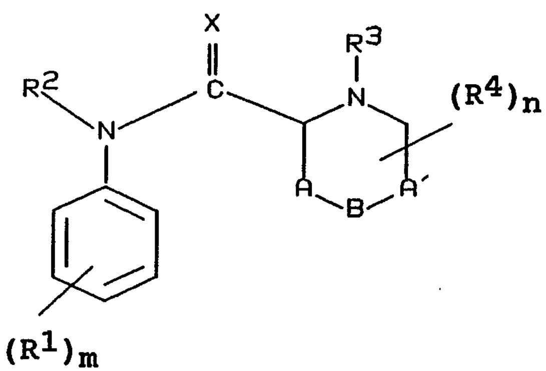

| Beispiel | $(R^1)_m$ | $R^2$ | $R^3$ | $(R^4)_n$ | A | B | A' | X | Fp [°C] |
|---|---|---|---|---|---|---|---|---|---|
| 3 | 4-Cl | H | $CH_3CO-$ | - | $CH_2$ | $CH_2$ | $CH_2$ | O | 170 |
| 4 | 2-F, 4-Cl, 5-$OCH_3$ | " | " | - | " | " | " | " | Öl |
| 5 | 4-Cl | " | $C_2H_5OOC-$ | - | " | " | " | " | " |
| 6 | 2-F, 4-Cl, 5-$OCH_3$ | " | $C_6H_5CH_2OOC-$ | - | " | " | " | " | " |
| 7 | 4-Cl | " | " | - | " | " | " | " | " |
| 8 | 4-Cl | " | 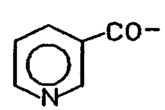 | - | " | " | " | " | 60-62 |
| 9 | 4-Cl | " | 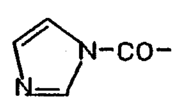 | - | " | " | " | " | 110-115 |

Fortsetzung Tabelle 1 Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 10 | 4-Cl | H | $4-NO_2-C_6H_4CO-$ | - | $CH_2$ | $CH_2$ | $CH_2$ | O | 180-185 |
| 11 | 4-Cl | " | $C_6H_5CO-$ | - | " | " | " | " | 74-78 |
| 12 | 2-F, 4-Cl, 5-$OCH_3$ | " | $4-NO_2-C_6H_4CO-$ | - | " | " | " | " | 61-63 |
| 13 | 2-F, 4-Cl, 5-$OCH_3$ | " | $C_6H_5CO-$ | - | " | " | " | " | Öl |
| 14 | 4-$CF_3$ | " | $C_2H_5OOC-$ | - | " | " | " | " | " |
| 15 | 4-$CF_3$ | " | " | - | " | " | " | " | " |
| 16 | 4-Cl | " | 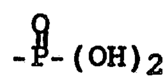 | - | " | " | " | " | |
| 17 | 2-F, 4-Cl, 5-$OCH_3$ | " | " | - | " | " | " | " | |
| 18 | 4-Cl | " | $C_6H_5-SO_2-$ | - | " | " | " | " | |
| 19 | 2-F, 4-Cl, 5-$OCH_3$ | " | " | - | " | " | " | " | |
| 20 | 4-Cl | " | 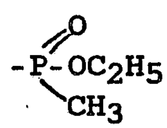 | - | " | " | " | " | |
| 21 | 2-F, 4-Cl, 5-$OCH_3$ | " | " | - | " | " | " | " | |

**Fortsetzung Tabelle 1** Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 22 | 4-Cl | H | 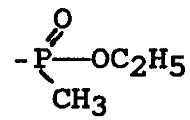 | - | $CH_2$ | $CH_2$ | $CH_2$ | O | |
| 23 | 2-F, 4-Cl, 5-$OCH_3$ | " | " | - | " | " | " | " | |
| 24 | 4-Cl | " | $C_6H_5$-SO- | - | " | " | " | " | |
| 25 | 2-F, 4-Cl, 5-$OCH_3$ | " | " | - | " | " | " | " | |
| 26 | 2-F, 4-Cl, 5-$OCH_3$ | " | $C_6H_5CH_2$ | - | " | " | " | " | |
| 27 | 2-F, 4-Cl, 5-$OCH_3$ | " | $CH_3OCH_2$- | - | " | " | " | " | |
| 28 | 4-Cl | " | $C_2H_5OOC$- | - | S | " | " | " | |
| 29 | 2-F, 4-Cl | " | " | - | S | " | " | " | |
| 30 | 4-Cl | " | " | - | $CH_2$ | S | " | " | |
| 31 | 2-F, 4-Cl, 5-$OCH_3$ | " | " | - | " | S | " | " | |
| 32 | 4-Cl | " | " | - | CH-$CH_3$ | O | CH-$CH_3$ | " | |
| 33 | 2-F, 4-Cl, 5-$OCH_3$ | " | " | - | $CH_2$ | O | $CH_2$ | " | |

Fortsetzung Tabelle 1    Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 34 | 4-Cl | H | $C_2H_5OOC-$ | - | $CH_2$ | $NCH_2C_6H_5$ | $CH_2$ | O | |
| 35 | 2-F, 4-Cl, 5-$OCH_3$ | " | " | - | " | " | " | " | |
| 36 | " " " | " | " | - | " | $CH-CH_3$ | " | " | |
| 37 | " " " | " | " | 2,6-$(CH_3)_2$ | " | O | " | " | |
| 38 | " " " | " | $-\overset{\overset{O}{\|}}{P}(OC_2H_5)_2$ | - | " | $CH_2$ | " | " | |
| 39 | " " " | " | $-\overset{\overset{O}{\|}}{P}(OH)_2$ | - | " | " | " | " | |
| 40 | " " " | " | $-\underset{\underset{O}{\|}}{\overset{\overset{O}{\|}}{S}}-OCH_3$ | - | " | " | " | " | |
| 41 | " " " | " | $-SO_3H$ | - | " | " | " | " | |
| 42 | " " " | " | $-\overset{\overset{O}{\|}}{S}-OCH_3$ | - | " | " | " | " | |
| 43 | 3-$COOCH(CH_3)COOC_2H_5$ 4-Cl 5-$OCH_3$ | " | $C_6H_5CH_2OOC-$ | - | " | " | " | " | |
| 44 | " " " | " | $C_2H_5OOC-$ | - | " | " | " | " | |

<u>Fortsetzung Tabelle 1</u> Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 45 | 4-Cl | H | $-SO_2-CH_3$ | - | $CH_2$ | $CH_2$ | $CH_2$ | O | |
| 46 | 2-F, 4-Cl, 5-$OCH_3$ | " | " | - | " | " | " | " | |
| 47 | 4-Cl | " | $-SO_2-OCH_3$ | - | " | " | " | " | |
| 48 | 2-F, 4-Cl, 5-$OCH_3$ | " | t-$C_4H_9OOC$- | - | " | " | " | " | |
| 49 | 4-Cl | " | " | - | " | " | " | " | |
| 50 | 4-Cl | $(CH_3)_2CH$ | $C_2H_5OOC$- | - | " | " | " | " | Öl |
| 51 | 4-Cl | H | $(CH_3)_2N-SO_2$- | - | " | " | " | " | 146-148 |
| 52 | 2-F, 4-Cl, 5-$OCH_3$ | " | " | - | " | " | " | " | |
| 53 | 4-Cl | " | $C_2H_5OOC-CO$- | - | " | " | " | " | 146-148 |
| 54 | 2-F, 4-Cl, 5-$OCH_3$ | " | " | - | " | " | " | " | |
| 55 | 4-Cl | " | $CH_3OOC$- | - | " | " | S | " | |
| 56 | 2-F, 4-Cl, 5-$OCH_3$ | " | " | - | " | " | S | " | |
| 57 | 4-Cl | " | $C_6H_5CH_2OOC$- | - | " | S | $CH_2$ | " | |
| 58 | 2-F, 4-Cl, 5-$OCH_3$ | " | " | - | " | S | " | " | |
| 59 | 3-$COOCH_2CF_3$, 4-Cl | " | " | - | " | $CH_2$ | " | " | |

Fortsetzung Tabelle 1    Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 60 | 3-$COOCH_2CH_2OH$, 4-Cl | H | $C_6H_5CH_2OOC$- | - | $CH_2$ | $CH_2$ | $CH_2$ | O | |
| 61 | 3-$COOC_2H_5$, 4-$OC_2H_5$ | " | " | - | " | " | " | " | |
| 62 | 3-$COOC_2H_5$, 4-$OCF_2CHF_2$ | " | " | - | " | " | " | " | |
| 63 | 2-F, 4-Cl, 5-$OCH_2$-C≡CH | " | " | - | " | " | " | " | |
| 64 | 4-Cl | " | H | - | " | " | " | " | 108-110 |
| 65 | 2-F, 4-Cl, 5-$OCH_3$ | " | " | - | " | " | " | " | 143-145 |
| 66 | " " " | " | $CH_3$ | - | " | " | " | " | |
| 67 | " " " | " | $C_2H_5OOC$-$CH_2$- | - | " | " | " | " | |
| 68 | " " " | " | $C_2H_5CH_2OOC$- | - | " | SO | " | " | |
| 69 | " " " | " | " | - | " | $SO_2$ | " | " | |
| 70 | " " " | " | $-\overset{O}{\overset{\|}{P}}(SC_2H_5)_2$ | - | " | $CH_2$ | " | " | |
| 71 | " " " | " | $C_6H_5OCH_2$- | - | " | " | " | " | |

Fortsetzung Tabelle 1 Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 72 | 2-F, 4-Cl, 5-$OCH_3$ | H | $CO_2C_2H_5$ | - | $CH_2$ | $CH_2$ | $CH_2$ | O | Öl |
| 73 | " | " | $CO_2CH_3$ | - | " | " | " | " | 125-128 |
| 74 | " | " | $CO_2n\text{-}C_8H_{17}$ | - | " | " | " | " | Öl |
| 75 | " | $CH_2OH$ | $CO_2CH_3$ | - | " | " | " | " | 123-126 |
| 76 | " | H | $CONHn\text{-}C_4H_9$ | - | " | " | " | " | 153 |
| 77 | " | $CH_3$ | $CO_2C_6H_5$ | - | " | " | " | " | 120-122 |
| 78 | " | " | $CO_2CH_2C_6H_5$ | - | " | " | " | " | Öl |
| 79 | " | " | $CO_2CH_3$ | - | " | " | " | " | " |
| 80 | " | " | $CO_2C_2H_5$ | - | " | " | " | " | " |
| 81 | " | " | $CH(CH_3)\text{-}CO_2C_2H_5$ | - | " | " | " | " | " |
| 82 | " | " | 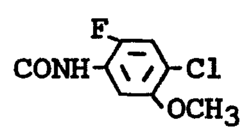 | - | " | " | " | " | " |

Fortsetzung Tabelle 1 Verbindungen der Formel I

| Beispiel | $(R^1)_m$ | $R^2$ | $R^3$ | $(R^4)_n$ | A | B | A' | X | Fp [°C] |
|---|---|---|---|---|---|---|---|---|---|
| 83 | 2-F, 4-Cl, 5-$OCH_3$ | H | $CONHC_6H_5$ | - | $CH_2$ | $CH_2$ | $CH_2$ | O | 164-165 |
| 84 | " | " | $CO_2$-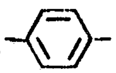-$NO_2$ | - | " | " | " | " | Öl |
| 85 | 2-F, 4-Cl, 5-$OCH_2C{\equiv}CH$ | " | $CO_2CH_3$ | - | " | " | " | " | " |
| 86 | " | " | $CO_2C_2H_5$ | - | " | " | " | " | " |
| 87 | 2-F, 4-Cl, 5-$OCH_3$ | H | $COCH_2Cl$ | - | $CH_2$ | $CH_2$ | $CH_2$ | O | Öl |
| 88 | " | " | $CONHC_2H_5$ | - | " | " | " | " | 170-172 |
| 89 | " | " | $CSN(C_2H_5)_2$ | - | " | " | " | " | Öl |
| 90 | " | " | $CON(C_2H_5)_2$ | - | " | " | " | " | " |
| 91 | " | " | $CO_2n$-$C_4H_9$ | - | " | " | " | " | " |
| 92 | " | " | $COCH_2P(O)(OC_2H_5)_2$ | - | " | " | " | " | " |
| 93 | " | " | $CH_2CO_2C_2H_5$ | - | " | " | " | " | " |
| 94 | " | " | 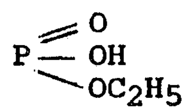 | - | " | " | " | " | " |
| 95 | " | " | CO-$CO_2C_2H_5$ | - | " | " | " | " | " |
| 96 | " | " | $CO_2C_6H_5$ | - | " | " | " | " | Öl |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 50 % Wirkung bzw. Schaden
4 = 60 - 80 % Wirkung bzw. Schaden
5 = 80 - 100 % Wirkung bzw. Schaden

**1. Unkrautwirkurg im Vorauflauf**

Samen bzw. Rhiozomstücke von mono-und dikotylen Unkrautpflanzen wurden in Plastiktöpfen (Ø = 9 cm) in sandiger Lehmerde ausgelegt und mit Erde abgedeckt. Die in Form von benetzbaren Pulvern von Emulsionskonzentraten formulierten erfindungsgemäßen Verbindungen wurden dann als wäßrige Suspensionen bzw. Emulsionen mit einer Wasseraufwandmenge von umgerechnet 600 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkrautpflanzen gehalten (Temperatur 23 plus/minus 1°C, relative Luftfeuchte 60 - 80 %.

Die optische Bonitur der Pflanzen bzw. Auflaufschäden erfolgte nach dem Auflaufen der Versuchspflanzen nach einer Versuchszeit von 3 - 4 Wochen im Vergleich zu unbehandelten Kontrollen. Die Ergebnisse sind in Tabelle 2 zusammengestellt.

**2. Unkrautwirkung im Nachauflauf**

Samen bzw. Rhizomstücke von mono-und dikotylen Unkräutern wurden in Plastiktöpfen (Ø= 9 cm) in sandigem Lehmtoden ausgelegt, mit Erde abgedeckt und im Gewächshaus unter guten Wachstumsbedingungen angezogen. Drei Wochen nach Aussaat wurden die Versuchspflanzen im Dreiblattstadium behandelt.

Die als Spritzpulver bzw. als Emulsionskonzentrat formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 - 4 Wochen Standzeit der Versuchspflanzen im Gewächshaus unter optimalen Wachstumsbedingungen (Temperatur 23 plus/minus 1°C, relative Luftfeuchte 60 - 80 %) die Wirkung der Präparate optisch im Vergleich zu unbehandelten Kontrollen bonitiert. Die Ergebnisse sind in Tabelle 3 aufgelistet.

Tabelle 2: Vorauflaufwirkung

| Verbindung Beispiel Nr. | Dosis kg a.i./ha | herbizide Wirkung STM | SIA | LOM | ECG |
|---|---|---|---|---|---|
| 72 | 2,5 | 5 | 5 | 5 | 5 |
| 6 | 2,5 | 5 | 5 | 5 | 5 |
| 73 | 2,5 | 5 | 5 | 5 | 5 |
| 74 | 2,5 | 5 | 5 | 5 | 5 |
| 75 | 2,5 | 5 | 5 | 5 | 5 |

**Tabelle 3: Nachauflaufwirkung**

| Verbindung Beispiel Nr. | Dosis kg a.i./ha | herbizide Wirkung STM | SIA | LOM | ECG |
|---|---|---|---|---|---|
| 72 | 2,5 | 5 | 5 | 5 | 4 |
| 6 | 2,5 | 3 | 5 | 3 | 2 |
| 73 | 2,5 | 3 | 2 | 3 | 2 |
| 75 | 2,5 | 5 | 5 | 4 | 3 |

Abkürzungen:

STM = Stellaria media
SIA = Sinapis alba
LOM = Lolium multiflorum
ECG = Echinochloa crus-galli
a.i.= Aktivsubstanz

**Ansprüche**

1. Herbizide Mittel, dadurch gekennzeichnet, daß sie eine oder mehrere Verbindungen der Formel I oder deren Salze

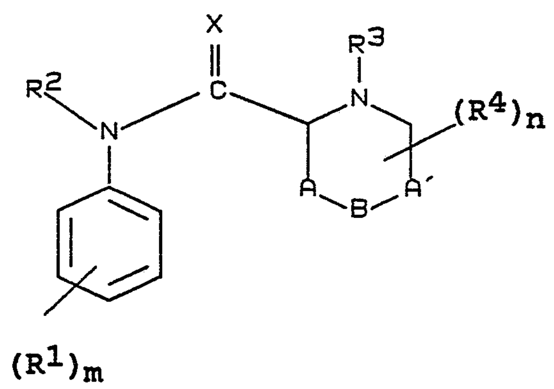

(I)

worin

A,A' = unabhängig voneinander $CH_2$, S, oder einer der beiden Reste A, A' = $CHR^4$, $C(R^4)_2$, >SO oder >$SO_2$,

B = O, S,>SO,>$SO_2$, $CH_2$, $CHR^4$, $C(R^4)_2$,>$NR^5$,>N-C=X($R^5$) oder>N-C=X($XR^5$), mit der Maßgabe, daß A und B oder A' und B nicht gleichzeitig O, S, SO oder $SO_2$ bedeuten dürfen,

X = O oder S,

$R_1$ = Halogen, $NO_2$, ($C_1$-$C_4$)Alkyl, -X($C_1$-$C_4$)Alkyl oder ($C_1$-$C_8$)Alkoxycarbonyl, wobei die letzten drei Reste bis zu sechsfach durch Halogen oder ein-oder zweifach durch OH substituiert sein können, ($C_3$-$C_6$)Cycloalkyl, ($C_3$-$C_6$)-Alkenyl, ($C_5$-$C_6$)-Cycloalkenyl, ($C_3$-$C_7$)Alkinyl, ($C_1$-$C_4$)Alkoxy($C_1$-$C_4$)alkyl, ($C_1$-$C_4$)Alkoxy($C_1$-$C_4$)alkoxycarbonyl, ($C_1$-$C_8$)Alkoxycarbonyl($C_1$-$C_8$)-alkoxycarbonyl, ($C_1$-$C_8$)Alkoxycarbonylmethylamino, -XH, -X($C_3$-$C_6$)Cycloalkyl, -X($C_3$-$C_6$)Alkenyl, -X($C_5$-$C_6$)-Cycloalkenyl, -X($C_3$-$C_7$)Alkinyl, -X[($C_1$-$C_4$)Alkoxy ($C_1$-$C_4$)alkyl] oder -X[($C_1$-$C_8$)Alkoxycarbonyl],

$R^2$ = H, ($C_1$-$C_4$)Alkyl oder ($C_1$-$C_4$)Hydroxyalkyl,

$R^3$ = H, ($C_1$-$C_6$)Alkyl, ($C_3$-$C_6$)Cycloalkyl, ($C_1$-$C_4$)Haloalkyl, ($C_1$-$C_4$)Alkoxy($C_1$-$C_4$)alkyl, ($C_1$-$C_4$)-Alkoxycarbonylmethyl, Phenoxy($C_1$-$C_4$)alkyl, Nicotinoyl, 1-Imidazolylcarbonyl, die Bedeutung von $R^5$ oder einen Rest der Formel

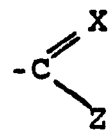

oder 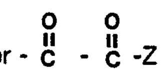 ,

worin

Z= die Bedeutung von $R^5$ besitzt oder den Rest $OR^5$, Halogen($C_1$-$C_4$)alkyl, ($C_1$-$C_4$)Alkoxy($C_1$-$C_4$)alkyl, Phenoxy($C_1$-$C_4$)alkyl, ($C_1$-$C_4$)Alkylamino oder Phenylamino, worin der Phenylrest durch Halogen, ($C_1$-$C_4$)-Alkoxy oder ($C_1$-$C_4$)Alkyl substituiert sein kann, bedeutet,

ferner einen Rest der Formeln

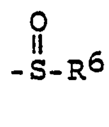 , 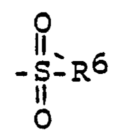 , 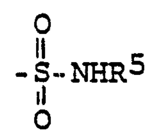 , 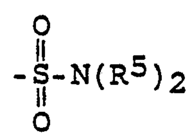 ,

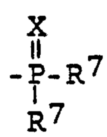 oder 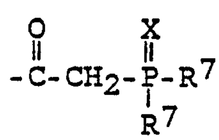 ,

$R^4$ = unabhängig voneinander die Bedeutung von $R^5$, -C=X($R^5$) oder -C=X($XR^5$),

$R^5$ = Phenyl, Pyridyl oder Benzyl,die bis zu dreifach durch ($C_1$-$C_4$)Alkyl, ($C_1$-$C_4$)Alkoxy, Halogen, $CF_3$ oder $NO_2$ substituiert sein können, oder ($C_1$-$C_8$)Alkyl,

$R^6$ = unabhängig voneinander H, Halogen, OH, die Bedeutung von $R^5$ oder den Rest $OR^5$,

$R^7$ = die Bedeutung von $R^6$ sowie SH oder den Rest $SR^5$,

m = 0, 1, 2, 3, 4 oder 5

n = 0, 1, 2 oder 3

bedeuten, als Wirkstoff(e) enthalten.

2. Herbizide Mittel gemäß Anspruch 1, dadurch gekennzeichnet, daß A, A', B = $CH_2$; X = O; $R^1$ = Halogen, ($C_1$-$C_4$)Alkoxy, ($C_3$-$C_7$)Alkinyloxy oder ($C_1$-$C_8$)Alkoxycarbonyl($C_1$-$C_8$)alkoxycarbonyl, $R^2$ = H, ($C_1$-$C_4$)-Alkyl oder $CH_2OH$; $R^3$ = ($C_1$-$C_4$)Alkoxycarbonyl, ($C_1$-$C_4$)Alkoxysulfonyl oder Di($C_1$-$C_4$)Alkoxyphosphoryl; m = 1, 2 oder 3 und n = 0 bedeuten.

3. Verbindungen der Formel I von Anspruch 1 oder deren Salze mit Ausnahme der Verbindungen der Formel I, worin

a) A, B und A' = $CH_2$; X = O; $R^1$ = Chlor, Methyl, Ethyl, Ethoxy, n-Butoxy; $R^2$ = H; $R^3$ = Methyl, Ethyl, n-Propyl n-Butyl, $(R^4)_n$ = H und m = 0, 1, 2 oder 3;

b) A, B und A' = $CH_2$; X = O; $R^2$ = H oder $C_2H_5$; $R^3$ = $CH_3$ oder $CO_2CH_3$; $(R^4)_n$ = H oder mono-$CH_3$ und m = O;

c) A, B und A' = $CH_2$; X = O; $(R^1)_m$ = 4-Chlor; $R^2$ = H; $R^3$ = nicotinoyl und $(R^4)_n$ = H; und

d) A und A' = $CH_2$; B = O; X = O; $(R^1)_m$ = H; $R^2$ = H; $R^3$ = $CO_2CH_3$ und $(R^4)_n$ = H

bedeuten.

4. Verfahren zur Herstellung von Verbindungen der Formel I oder deren Salze gemäß Anspruch 3, dadurch gekennzeichnet, daß man ein α-Iminocarbonsäureanilid der Formel II

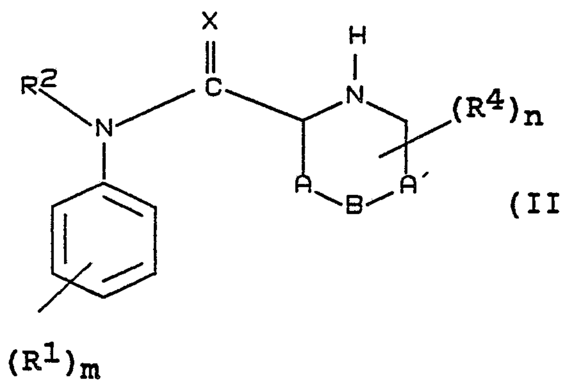

(II)

$R^3$-Hal

(III)

mit einem Halogenid der Formel III in Gegenwart einer Base umsetzt und die erhaltenen Verbindungen gegebenenfalls in ihre Salze umwandelt.

5. Verwendung von Verbindungen der Formel I gemäß Anspruch 1 oder 2 als Herbizide.

6. Verfahren zur Bekämpfung von unerwünschten Pflanzen, dadurch gekennzeichnet, daß man eine wirksame Menge eines Mittels gemäß Anspruch 1 oder 2 auf die zu behandelnden Anbauflächen oder die zu behandelnden Pflanzen aufbringt.